# EUROPÄISCHE PATENTSCHRIFT

(11) **EP 1 611 870 B1**
(45) Veröffentlichungstag und Bekanntmachung des Hinweises auf die Patenterteilung: **15.10.2008**
(21) Anmeldenummer: 05008328.6
(22) Anmeldetag: 16.04.2005
(51) Int. Cl.: A61F 2/36, A61F 2/46

(54) **Prüfhilfe bei der Versorgung eines präparierten Restgelenkkopfes eines Hüftgelenks**
Testing device used in the preparation of a partially ablated femoral head
Dispositif d'essai utilisé dans la préparation d'une tête fémorale partiellement enlevée

(30) Priorität: 02.07.2004 DE 102004032675
(43) Veröffentlichungstag der Anmeldung: 04.01.2006
(73) Patentinhaber: ESKA Implants GmbH & Co. KG, 23556 Lübeck (DE)
(72) Erfinder: Grundei, Hans, Dr., 23556 Lübeck (DE)
(74) Vertreter: Fuchs

(56) Entgegenhaltungen:
- EP-A- 1 477 120
- US-A- 6 156 069
- US-A1- 2003 018 391

## Beschreibung

Die vorliegende Erfindung betrifft eine Prüfhilfe bei der Versorgung eines präparierten Restgelenkkopfes mit einem der Form einer Gelenkkugel eines Hüftgelenks nachgebildeten Kappenimplantat.

Bei Schädigungen im Hüftgelenksbereich ist man bemüht, soviel natürliches Knochenmaterial zu erhalten wie möglich. Daher erscheint es bei relativ kleinen Schädigungen, wie etwa einer Hüftgelenkskopfnekrose, als unangemessen, durch Resektionen großer Bereiche einen Ersatz für die dann resezierte Gelenkkugel zu schaffen. Dabei kommen dann üblicherweise sogenannte Langstielendoprothesen zum Einsatz. Bei derartigen Schäden, die den Patienten gleichwohl beeinträchtigen, kommen aber auch zunehmend sogenannte Schenkelhalsendoprothesen zum Einsatz, bei denen der Prothesenstiel lediglich in der Metaphyse und der Diaphyse liegt. Der Einsatz eines derartigen Implantates, wie es beispielsweise bekannt ist aus der EP 0 878 176 A2, gestattet, im Falle von Komplikationen nach langer Tragezeit den Rückzug auf die schon genannten und bekannten Langstielendoprothesen. Bei der Implantation der Schenkelhalsendoprothese wird von vornherein eine geringere Resektion des Schenkelhalses vorgenommen als dies bei der Implantation einer Langstielendoprothese der Fall ist. Im Falle des notwendig werdenden Austausches kann nun der Rest des Schenkelhalses reseziert werden in einem Maße, wie es von vorneherein bei der Implantation einer Langstielendoprothese angezeigt gewesen wäre, wodurch die endoprothetische Versorgung des Patienten um einen Zyklus verlängert werden kann.

Schädigungen wie eine Kopfnekrose rechtfertigen aber selbst eine so vorsichtige Vorgehensweise nicht immer. Um die Resektion bei einer Implantation auf ein Mindestmaß zu reduzieren, ist in der DE 102 18 801 B3 ein Kappenimplantat vorgeschlagen worden. Dieses besteht im wesentlichen aus einer der Form der Gelenkkugel nachgebildeten Kappe mit einem angeformten, vom Polbereich zur konkaven offenen Seite abragenden Steckkonus und einer im Inneren der Kappe umlaufenden Fixationshilfe. Die Kappe wird auf den präparierten Restgelenkkopf gesetzt.

Ein derartiges Kappenimplantat kommt in jüngerer Zeit verstärkt zur Anwendung. Vorraussetzung für den Einsatz eines solchen Kappenimplantates ist die sorgfältige Vorbereitung des natürlichen Restgelenkkopfes, damit dann eine möglichst weitgehende Übereinstimmung zwischen der Kopfbelastungsachse und der Achse der Kappe erzielt wird. Darüber hinaus sind der CCD- und der Antekurvationswinkel so einzustellen, daß diese weitgehend den Werten in natürlichen Hüftgelenken entsprechen.

Erschwerend kommt hinzu, daß sich beispielsweise bei einer Nekrose der Gelenkkopf verlagert. Meist ist dies in Richtung caudal der Fall. Aber auch nach medial oder lateral sind Verschiebungen durchaus möglich, abhängig vom jeweiligen Krankheitsbild. In einem solchen Fall das Kappenimplantat so einzustellen, daß sich praktisch natürliche Winkel- und Lageverhältnisse ergeben, ist relativ schwierig. Bislang war dies nur erfahrenen Operateuren vorbehalten. Allerdings kommt es immer wieder zu Fehlstellungen des Kappenimplantates nach erfolgter Implantation. Aus diesem Grunde ist in der DE 102 61 813 A eine Zentrierhilfe für ein Gelenkkopf-Kappenimplantat eines künstlichen Hüftgelenkes vorgeschlagen worden, mittels der das anatomische Polzentrum des Hüftgelenkkopfes aufgefunden werden kann.

Nach dem Auffinden des anatomischen Polzentrums wird der Restgelenkkopf präpariert, in der Weise, daß die natürliche Gelenkkugel teilreseziert wird, so daß ein Restgelenkkopf zurückbleibt, über den dann das eingangs erwähnte Kappenimplantat gesetzt werden kann.

Wenn dieses System auch sehr zufriedenstellende Ergebnisse ermöglicht hat, ist aus Kreisen der Operateure der Wunsch geäußert worden, zwischen dem Schritt der Teilresektion des Gelenkkopfes und dem Aufsetzen des Kappenimplantates eine Überprüfungsmöglichkeit bereitzustellen, die es ermöglicht abzuschätzen, wie der Sitz des Kappenimplantates auf dem Restgelenkkopf voraussichtlich sein wird. Die Anwendung der erwähnten Zentrierhilfe nämlich ist immer noch ein empirisches Verfahren, keineswegs ein mathematisch exaktes Verfahren, so daß trotz aller Sorgfalt noch Abweichungen vom Optimum auftreten können, die in einem Zwischenschritt dann noch korrigiert werden können.

Die US-Patentschrift US-A-6,156,069 offenbart eine Prüfhilfe zur Versorgung eines Restgelenkkopfes, allerdings besteht dort kein Formenschluss.

Demgemäß ist es die Aufgabe der vorliegenden Erfindung, eine derartige Vorrichtung oder ein derartiges Gerät vorzuschlagen.

Gelöst wird diese Aufgabe durch eine Prüfhilfe bei der Versorgung eines präparierten Restgelenkkopfes mit einem der Form einer Gelenkkugel eines Hüftgelenks nachgebildeten Kappenimplantat, mit den Merkmalen des Anspruchs 1. Vorteilhafte Weiterbildungen ergeben sich aus den Unteransprüchen.

Demgemäß dient die Prüfhilfe zur Überprüfung des Formenschlusses zwischen dem Restgelenkkopf und der kappenförmigen Prüfhilfe selbst. Deren Inneres gleicht weitgehend dem Inneren des Kappenimplantates, welches nach erfolgreicher Überprüfung des Formenschlusses auf den Restgelenkkopf gesetzt werden soll. Zur Prüfung des Formenschlusses weist die Prüfhilfe eine Oberfläche auf, welche durch wenigstens 2 Bohrungen durchsetzt ist. Die Bohrungen dienen zur optischen und/oder taktilen Kontrolle des Formenschlusses zwischen ihr und dem Restgelenkkopf. Der Operateur kann also durch die Durchbohrungen hindurch sehen, inwieweit der Restgelenkkopf eng an der Innenoberfläche der Prüfhilfe anliegt. Zusätzlich oder alternativ kann er die Anlage des Restgelenkkopfes an dem Inneren der Prüfhilfe ertasten. Sollte sich bei der Überprüfung herausstellen, daß der Formenschluss zwischen Restgelenkkopf und der Prüfhilfe nicht optimal ist bzw. nicht den Vorstellungen des Operateurs entspricht, so kann dieser durch erneute Anwendung eines Präparationsinstrumentes, beispielsweise eines Fräsers nachhelfen, solange bis das Ergebnis zufriedenstellend ist. Das Ergebnis der Präparation und der Überprüfung mittels der erfindungsgemäßen Prüfhilfe ist ein Restgelenkkopf mit einem anatomischen Polzentrum (DE 102 61 813 A) und mit einer Außenkontur, welche einen optimalen Formenschluss mit dem letztendlichen Kappenimplantat eingeht. Betont werden muss an dieser Stelle, daß das Innere der kappenförmigen Prüfhilfe praktisch identisch sein muss mit dem Inneren des letztendlichen Kappenimplantates. Betont werden muss an dieser Stelle ebenfalls, daß die Prüfhilfe kein Implantat ist, sondern lediglich ein Hilfsmittel zur Optimierung des besagten Formenschlusses.

In besonderer Ausgestaltung ist vorgesehen, daß die Prüfhilfe einen im Inneren von ihrem Polbereich abragenden, in eine entsprechende Bohrung im Restgelenkkopf setzbaren Führungszapfen aufweist. Dies erlaubt eine definierte Positionsbestimmung der Prüfhilfe auf dem Restgelenkkopf.

Gemäß einer noch weiteren besonderen Ausführungsform ist in der Basiskante der Prüfhilfe wenigstens eine Markierungskerbe vorgesehen. Diese Markierungskerbe gestattet die Anbringung einer Markierung auf dem Restgelenkkopf, bis zu der das letztendliche Kappenimplantat mit seiner Basiskante aufgesetzt werden muss, um dieselbe Lage auf dem Restgelenkkopf einzunehmen wie die Prüfhilfe. In besonderer Anwendung zusammen mit dem erwähnten Führungszapfen kann der Operateur einen geeigneten Stift in die Markierungskerbe setzen und die Prüfhilfe, sofern sie ein rotationssymmetrisches Inneres aufweist, drehend um den Restgelenkkopf führen, so daß eine Linie erzeugt wird. Das letztendliche Kappenimplantat wird dann so auf den Restgelenkkopf gesetzt, daß seine Basiskante deckungsgleich ist mit der so erzeugten Linie auf dem Restgelenkkopf.

Da es sich bei der erfindungsgemäßen Prüfhilfe nicht um ein Implantat, sondern um ein Hilfsmittel handelt, ist es wiederverwendbar. Aus diesem Grunde besteht die Prüfhilfe in bevorzugter Ausführungsform aus einem sterilisierbaren Kunststoff. Die Sterilisierbarkeit ist geboten, um in verschiedenen Operationssitzungen eingesetzt werden zu können. Kunststoff als Material bietet sich aus Kostengründen und der guten Formbarkeit an. Als Material kommt freilich auch beispielsweise Metall in Betracht.

Gemäß einer noch weiteren vorteilhaften Ausführungsform ist im Polbereich der Prüfhilfe eine Gewindebohrung eingelassen. In diese Gewindebohrung ist nach abgeschlossener Überprüfung des Formenschlusses das Eindrehen eines Abzugsinstrumentes möglich, mittels dessen die Prüfhilfe von dem Restgelenkkopf abgezogen werden kann, damit entweder eine Nachbearbeitung des Restgelenkkopfes stattfinden kann oder aber das letztendliche Kappenimplantat auf den Stumpf gesetzt werden kann.

Die Erfindung wird anhand eines Ausführungsbeispiels gemäß den Zeichnungsfiguren näher erläutert. Hierbei zeigt:
- Fig. 1: die Aufsicht auf die Prüfhilfe,
- Fig. 2: schematisch einen Querschnitt durch die Prüfhilfe,
- Fig. 3: eine Innenansicht der Prüfhilfe, und
- Fig. 4: eine Seitenansicht der Prüfhilfe, teilweise geschnitten.

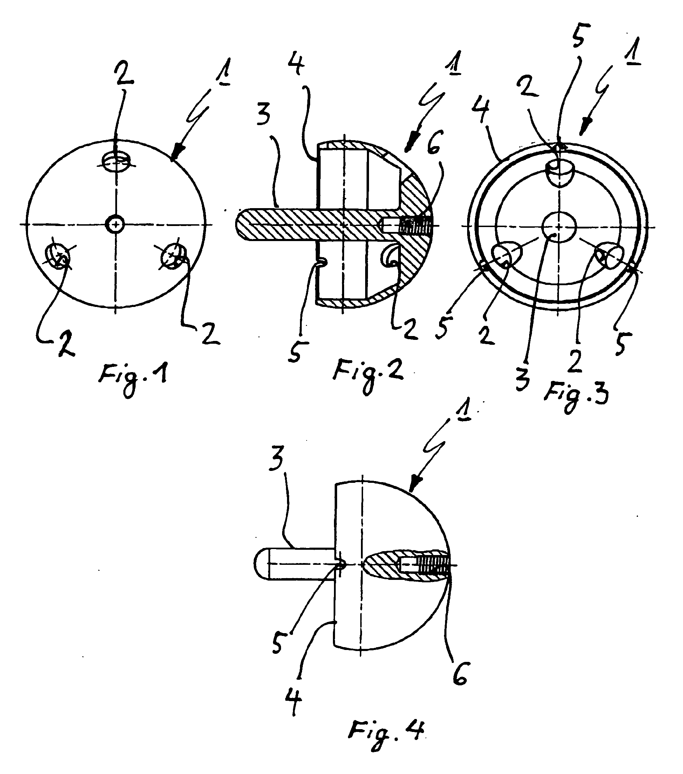

Fig. 1 zeigt eine Aufsicht auf die kalottenförmige Kappe 1 in Richtung ihres Poles P. Vorliegend sind in der Wandung der Kappe 1 drei Durchbohrungen 2 vorgesehen. Diese durchsetzen die Oberfläche der Kappe 1. Durch diese Durchbohrungen 2 kann der Operateur nach Aufsetzen der Prüfhilfe auf den Restgelenkkopf den Formenschluss zwischen diesem und der Prüfhilfe optisch kontrollieren oder ertasten, beispielsweise auch mit einem Führungsdraht.

Wie aus Fig. 2 ersichtlich weist die Prüfhilfe einen im Inneren vom Pol abragenden Führungszapfen 3 auf. Dieser ist in eine entsprechend im Restgelenkkopf anzubringende Bohrung setzbar, damit die Position der Prüfhilfe auf dem Restgelenkkopf definiert ist. In der Basiskante 4 der Kappe 1 sind in der abgebildeten Ausführungsform drei Markierungskerben 5 (Fig. 3) eingelassen. Diese Markierungskerben 5 dienen zum Einführen eines Markierungsstiftes (nicht dargestellt), um die Höhe zu markieren, bis zu der das letztendliche Kappenimplantat (nicht dargestellt) auf den Restgelenkkopf gesetzt werden muss, um eine entsprechende Lage auf dem Restgelenkkopf einzunehmen wie die Prüfhilfe selbst. Bei einer rotationssymmetrischen Ausbildung des Inneren der Kappe 1, wie in Fig. 2 dargestellt, kann nach Einführung des Führungszapfens in die schon erwähnte Bohrung im Restgelenkkopf die Kappe 1 bei in der Markierungskerbe eingesetztem Markierungsstift gedreht werden, um eine durchgehende Markierungslinie auf dem Restgelenkkopf zu erzeugen.

Im Polbereich P ist eine Gewindebohrung 6 eingelassen. Diese dient zum Einschrauben einer Abziehhilfe nach erfolgter Überprüfung des Formenschlusses zwischen der Prüfhilfe und dem Restgelenkkopf.

Die Fig. 3 und 4 dienen zur Abrundung des Bildes, wobei Fig. 3 eine Ansicht in das Innere der Kappe 1 darstellt. Deutlich erkennbar sind die drei Markierungskerben 5 in der Basiskante 4 der Kappe 1, sowie die drei Durchbohrungen 2 in ihrer Oberfläche. Fig. 4 zeigt dabei die Seitenansicht der Kappe 1, geschnitten im Bereich der Gewindebohrung 6 im Polbereich.

## Patentansprüche

1. Prüfhilfe bei der Versorgung eines präparierten Restgelenkkopfes eines Hüftgelenkes mit einem der Form einer Gelenkkugel eines Hüftgelenks nachgebildeten Kappenimplantat auf Formenschluss mit dem Restgelenkkopf in Form einer über den Restgelenkkopf setzbaren Kappe (1), deren Inneres weitgehend dem Inneren des Kappenimplantates gleicht und deren Oberfläche durch wenigstens zwei Bohrungen (2) durchsetzt ist zur optischen und/oder taktilen Kontrolle des Formenschlusses zwischen ihr und dem Restgelenkkopf.

2. Prüfhilfe nach Anspruch 1, bei der im Inneren ein vom Polbereich abragender, in eine entsprechende Bohrung im Restgelenkkopf setzbarer Führungszapfen (3) vorgesehen ist.

3. Prüfhilfe nach Anspruch 1 oder 2, bei der in ihrer Basiskante (4) wenigstens eine Markierungskerbe (5) vorgesehen ist.

4. Prüfhilfe nach einem der Ansprüche 1 bis 3, die aus einem sterilisierbaren Kunststoff besteht.

5. Prüfhilfe nach einem der Ansprüche 1 bis 4, bei der im Polbereich eine Gewindebohrung (6) eingelassen ist.

## Claims

1. Test aid when supplying a dissected residual condyle of a hip joint with a cap implant copying the shape of a condyle of a hip joint mould clamping with the residual condyle in the form of a cap (1) which can be placed over the residual condyle, the interior of which cap (1) is largely similar to the interior of the cap implant and the surface of which is penetrated by at least two bores (2) for optical and/or tactile control of the mould clamping between it and the residual condyle.

2. Test aid according to claim 1, in which a guide pin (3) projecting from the pole region and which can be placed in a corresponding bore in the residual condyle is provided in the interior.

3. Test aid according to claim 1 or 2, in which at least one marking notch (5) is provided in its base edge (4).

4. Test aid according to one of claims 1 to 3, which consists of a plastic which can be sterilised.

5. Test aid according to one of claims 1 to 4, in which a threaded bore (6) is admitted in the pole region.

## Revendications

1. Dispositif d'essai utilisé dans la préparation d'une tête fémorale partiellement enlevée avec un implant en calotte, imitant la forme d'une articulation à rotule d'une tête fémorale, pour vérifier l'adaptation de forme avec la tête fémorale partiellement enlevée, en forme de calotte (1) pouvant être installée sur la tête fémorale partiellement enlevée, dont l'intérieur est globalement identique avec l'intérieur de l'implant en calotte et dont la surface externe est pénétrée par au moins deux trous (2) pour un contrôle optique et/ou tactile de l'adaptation de forme entre elle et la tête fémorale partiellement enlevée.

2. Dispositif d'essai selon la revendication 1, dans lequel, à l'intérieur, est prévue une tige de guidage (3) faisant saillie depuis la zone polaire et pouvant être installée dans un trou correspondant dans la tête fémorale.

3. Dispositif d'essai selon l'une des revendications 1 et 2, dans lequel il est prévu au moins une entaille de marquage (5) dans son bord de base (4).

4. Dispositif d'essai selon l'une des revendications 1 à 3, qui est formé de matière plastique pouvant être stérilisée.

5. Dispositif d'essai selon l'une des revendications 1 à 4, dans lequel un trou taraudé (6) est ménagé dans la zone polaire.
